# EUROPEAN PATENT APPLICATION

(11) **EP 4 813 422 A1**
(43) Date of publication of application: **30.09.2026**
(21) Application number: 26168110.0
(22) Date of filing: 26.03.2026
(51) Int. Cl.: A61M 5/315

(54) **PRECISE DOSING SYRINGE ASSEMBLY**

(30) Priority: 26.03.2025 US 202563777994 P
(71) Applicant: Pacto Medical, Inc., Burlington, VT 05408 (US)
(72) Inventor: HALVORSEN Jr., George Robert, San Diego, CA, 92122 (US); STINEBAUGH, Ryan, Lawnside, NJ, 08003 (US); SPEERS, Ian, 05408 Burlington, VT (AS)
(74) Representative: Savi, Massimiliano

(57) **Abstract**

A dosing plunger system for a precise dosing syringe assembly includes a plunger having a plunger rod and a repositionable locking clip. The locking clip slides longitudinally along the plunger rod without rotation to a selected position and comprises a gripping portion configured to be manually actuated between a locked configuration and a released configuration, one or more engagement features that engage the plunger rod in the locked configuration to prevent longitudinal movement of the locking clip relative to the plunger rod, and a stop surface sized to extend beyond an inner diameter of a proximal barrel entrance. When the plunger is advanced distally, the stop surface contacts an outer rim of the proximal entrance, limiting plunger travel and establishing a maximum dispensable volume. The locking clip is repeatedly repositionable without rotation to accommodate patient-specific dosing. The system is compatible with both prefilled syringes and syringes filled by aspiration.

## Description

### CROSS-REFERENCE TO RELATED APPLICATION

This application claims the benefit of priority to U.S. Provisional Patent Application No. 63/777,994, filed March 26, 2025, entitled "Precise Syringe Dosing with Plunger-Mounted Lock Mechanism", the entire disclosure of which is incorporated herein by reference.

### FIELD

The present invention relates generally to medical devices for precise fluid delivery. More specifically, the invention relates to a dosing plunger system and syringe assembly configured to enable rapid, accurate dose preparation by limiting the maximum dispensable volume through a repositionable locking mechanism mounted on a syringe plunger rod.

### BACKGROUND

Accurate fluid (e.g., medication) dosing is critical in healthcare, particularly in emergency medical situations where rapid drug administration can be lifesaving. The challenge of precise dosing is especially acute where the appropriate dose varies significantly based on patient-specific parameters such as body weight, age, body surface area, disease severity, and other factors.

In emergency resuscitation scenarios, for example, the correct fluid dose for a small child may be only a fraction of the dose required for a larger child or adult. Administering an insufficient dose may render the treatment ineffective, while overdosing can cause severe harm or death. Healthcare providers must therefore calculate patient-specific doses quickly and accurately, often under time pressure and stressful conditions where the risk of human error is elevated.

Conventional approaches to precise dosing typically involve one or more of the following methods:

### Manual Calculation and Aspiration

Healthcare providers use measurement tools to assess patient size, perform dosing calculations based on weight or body surface area, and then manually draw the calculated dose from a fluid vial or ampoule into a standard syringe. The user (e.g., the healthcare provider or others including the patient themselves) must carefully observe volume markings on the syringe barrel to aspirate the correct amount. This process is time-consuming, requires multiple steps, and is prone to calculation errors, measurement errors, and improper reading of graduations, especially in high-stress emergency situations or low-light environments.

### Prefilled Syringes

Prefilled syringes contain a predetermined volume of fluid and eliminate the need for aspiration from a vial or ampoule. However, conventional prefilled syringes are manufactured with a fixed fluid volume and lack any mechanism to limit dispensing to a smaller, patient-specific dose. If a patient requires less than the full syringe contents, the provider must again rely on visual estimation using visual graduations to dispense only the required amount, reintroducing the risk of dosing error.

### Fixed-Dose Delivery Systems

Some existing syringe systems incorporate mechanisms to limit plunger rod travel and thereby control dispensed volume. However, these systems suffer from significant drawbacks. First, many prior art systems require twist-locking mechanisms or complex multi-component assemblies that involve rotating a locking element relative to a plunger rod to engage or disengage longitudinal positioning features. Such rotation-based systems are mechanically intricate, often require multiple hands to operate, and are cumbersome to adjust rapidly. Next, certain prior art locking mechanisms are designed primarily to prevent syringe reuse rather than to enable pre-dispensing dose adjustment. These mechanisms engage after partial or complete dispensing and do not allow for pre-setting a maximum dispensable volume before fluid is dispensed. Some systems provide discrete dose settings but lack the flexibility to accommodate a wide range of clinically relevant volume increments or to be easily repositioned along the plunger rod to correspond with different patient needs.

As such, there remains a significant unmet need in the medical device field for a syringe dosing system that: (1) enables rapid, intuitive adjustment of maximum dispensable volume without requiring rotational locking or complex manipulation; (2) allows healthcare providers to pre-set a dose limit on a prefilled syringe or an aspirated syringe before dispensing, thereby preventing accidental overdosing; (3) provides a simple, ergonomic mechanism that can be operated quickly, even in high-stress emergency situations; (4) accommodates a range of clinically relevant dose increments corresponding to patient-specific parameters such as weight, age, or body surface area; (5) maintains secure positioning of the dose-limiting mechanism during fluid dispensing to ensure the pre-set dose is reliably administered; (6) is compatible with standard syringe components and manufacturing processes to facilitate regulatory approval, widespread adoption and use with both prefilled syringes and conventional aspiration-based dosing workflows, and wherein the dose-limiting mechanism does not contact or alter any fluid-contacting components of the syringe assembly, thereby reducing regulatory burdens and simplifying the path to approval compared to prior art systems that modify fluid-contacting components; (7) permits rapid repositioning of the dose-limiting mechanism both to correct initial positioning errors and to administer additional medication when therapeutic effect is not achieved with an initial dose, in either case without requiring a new syringe or complex recalibration; and (8) provides tactile feedback through a physical stop mechanism to confirm that the intended dose volume has been fully dispensed, eliminating reliance on visual estimation or electronic indicators.

The present invention addresses these needs by providing a dosing plunger system and syringe assembly incorporating a repositionable locking clip that slides longitudinally along a plunger rod without rotation, engages the plunger rod through one or more engagement features that transition between locked and released configurations, and limits plunger rod travel by abutting an outer rim of the syringe barrel when the plunger rod is advanced distally during dispensing.

### NOTES ON CONSTRUCTION

The use of the terms "a", "an", "the" and similar terms in the context of describing the invention are to be construed to cover both the singular and the plural, unless otherwise indicated herein or clearly contradicted by context. The terms "comprising", "having", "including" and "containing" are to be construed as open-ended terms (i.e., meaning "including, but not limited to,") unless otherwise noted. The terms "substantially", "generally" and other words of degree are relative modifiers intended to indicate permissible variation from the characteristic so modified. The use of such terms in describing a physical or functional characteristic of the invention is not intended to limit such characteristic to the absolute value which the term modifies, but rather to provide an approximation of the value of such physical or functional characteristic.

Terms concerning attachments, coupling and the like, such as "connected" and "interconnected", refer to a relationship wherein structures are secured or attached to one another either directly or indirectly through intervening structures, as well as both moveable and rigid attachments or relationships, unless specified herein or clearly indicated by context. The term "operatively connected" is such an attachment, coupling or connection that allows the pertinent structures to operate as intended by virtue of that relationship.

The use of any and all examples or exemplary language (e.g., "such as" and "preferably") herein is intended merely to better illuminate the invention and the preferred embodiment thereof, and not to place a limitation on the scope of the invention. Nothing in the specification should be construed as indicating any element as essential to the practice of the invention unless so stated with specificity.

### BRIEF DESCRIPTION OF THE DRAWINGS

Further advantages of the invention are apparent by reference to the detailed description when considered in conjunction with the figures, which are not to scale so as to more clearly show the details, wherein like reference numerals represent like elements throughout the several views, and wherein:
**FIG. 1A** is a perspective view of a syringe assembly according to an embodiment of the present invention;
**FIG. 1B** is an exploded view of the syringe assembly of FIG. 1A;
**FIGS. 2A-2C** are rear perspective, front perspective, and left elevation views, respectively, of a plunger rod having a disc-shaped thumb press and visual indicators according to a first embodiment of the present invention;
**FIGS. 3A-3C** are rear perspective, front perspective, and left elevation views, respectively, of a plunger rod having a thumb press and visual indicators according to a second embodiment of the present invention;
**FIGS. 4A-4D** are rear perspective, front perspective, left elevation, and front elevation views, respectively, of a plunger rod having a thumb press configured to permit the plunger rod to nest alongside the syringe barrel and visual indicators according to a third embodiment of the present invention;
**FIG. 5A** is a front perspective view of a push-type locking clip according to a first embodiment of the present invention;
**FIG. 5B** and **FIG. 5C** are top and bottom plan views, respectively, of the locking clip shown in FIG. 5A;
**FIG. 5D** is a section view of the locking clip shown in FIG. 5B, taken along line "5D - 5D";
**FIG. 6A** and **FIG. 6B** are rear perspective and front perspective views, respectively, of a butterfly-type locking clip according to a second embodiment of the present invention;
**FIG. 6C** and **FIG. 6D** are rear elevation and front elevation views, respectively, of the locking clip of FIG. 6A;
**FIG. 6E** and **FIG. 6F** are top elevation and bottom elevation views, respectively, of the locking clip of FIG. 6A;
**FIG. 6G** is a sectional view of the locking clip of FIG. 6E, taken along line "6G -6G";
**FIG. 7A** and **FIG. 7B** are rear perspective and left elevation views, respectively, of a precise dosing syringe assembly employing the plunger rod shown in FIG. 2A and the locking clip shown in FIG. 5A in a released configuration;
**FIG. 7C** is a section view of the precise dosing syringe assembly shown in FIG. 7B, taken along line "7C -7C";
**FIG. 7D** is a section view of the precise dosing syringe assembly shown in FIG. 7C, taken along line "7D - 7D";
**FIG. 8A** and **FIG. 8B** are rear perspective and left elevation views, respectively, of a precise dosing syringe assembly employing a plunger rod according to the first embodiment having rounded indentations and the locking clip shown in FIG. 5A in a locked configuration;
**FIG. 8C** is a section view of the precise dosing syringe assembly shown in FIG. 8B, taken along line "8C -8C";
**FIG. 8D** is a section view of the precise dosing syringe assembly shown in FIG. 8C, taken along line "8D - 8D";
**FIG. 9A** provides a sectional view of a butterfly-type locking clip with flexible gripping portions that are not engaged with peripheral indentations of a plunger rod in a released configuration;
**FIG. 9B** depicts a front view of the locking clip and plunger rod of FIG. 9A, wherein a fluid volume indicator is located between a pair of visual indicators and is not aligned with either visual indicator;
**FIG. 10A** depicts the butterfly-type locking clip FIG. 9A but with the flexible gripping portions engaged with peripheral indentations of a plunger rod in a locked configuration; and
**FIG. 10B** depicts a front view of the locking clip and plunger rod of FIG. 10A, wherein a fluid volume indicator is located at one of the visual indicators.

### DETAILED DESCRIPTION

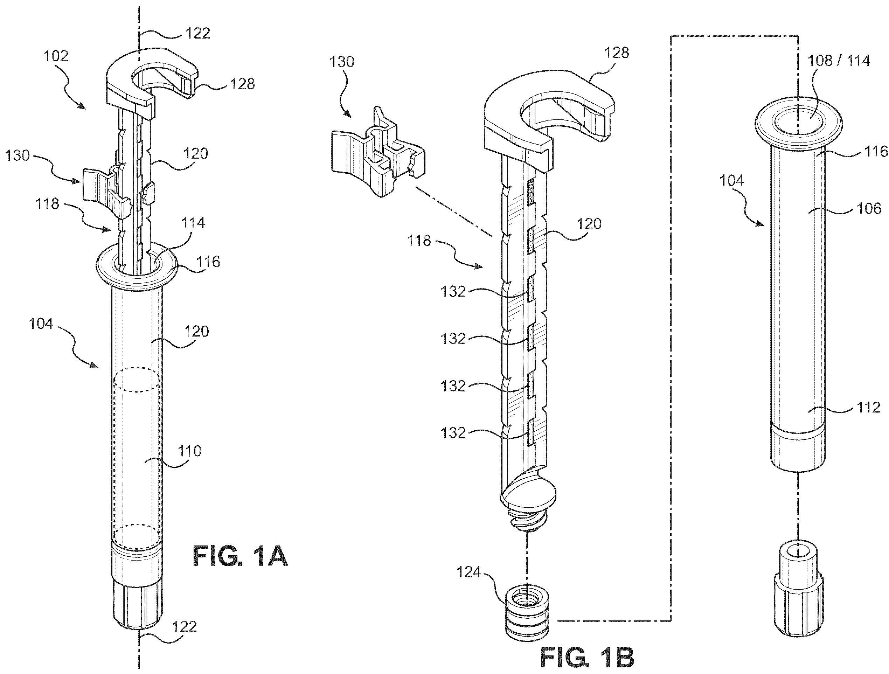

Referring now to the drawings in which like reference characters designate like or corresponding characters throughout the several views, there is shown in FIG. 1A and FIG. 1B a syringe assembly **102** configured to deliver precise, pre-set volumes of a fluid, which may be a medication or other fluids. The system is particularly well-suited for emergency medical applications, pediatric dosing, and any clinical scenario requiring rapid, accurate dose preparation based on patient-specific parameters.

### Overview of the Syringe Assembly

The syringe assembly 102 comprises a barrel **104** formed by a cylindrical body **106** defining an interior chamber **108** configured to contain a total volume of fluid **110.** The barrel 104 has a distal end **112** for dispensing the fluid 110 and a proximal entrance **114** having an outer rim **116.** In certain embodiments, the distal end 112 is configured with a standard Luer-lock or Luer-slip fitting to enable connection with a needle, catheter, or other fluid delivery component. The barrel 104 may be fabricated from a transparent or translucent material, such as medical-grade polypropylene, polycarbonate, or glass, to permit visual observation of the fluid 110 within the interior chamber 108.

A plunger **118** is provided, comprising a plunger rod **120** extending along a longitudinal axis **122** and configured to move within the barrel 104. The plunger 118 further includes a distal plunger seal **124** that forms a fluid-tight seal with an interior wall of the barrel 104, and a proximal thumb press **128** for manual actuation by a user. The plunger rod 120 may be formed from a rigid or semi-rigid medical-grade polymer, such as polypropylene or ABS, and the plunger seal 124 may be formed from a compliant elastomeric material, most commonly butyl rubber, though medical-grade silicone or thermoplastic elastomer may also be used, to maintain a fluid-tight seal throughout the range of plunger motion. In some embodiments, the plunger rod 120 and plunger seal 124 are formed as a single integrated component. In other embodiments, the plunger seal 124 is separately formed and is removably attached to a distal end of the plunger rod 120.

The syringe assembly 102 further comprises a locking clip **130** mounted on the plunger rod 120 between the thumb press 128 and the proximal entrance 114 of the barrel 104. As will be described in detail herein, the locking clip 130 is repositionable along the plunger rod 120 to a selected position, and serves as a physical stop that limits the maximum travel of the plunger 118 distally within the barrel 104, thereby establishing a precise, pre-set maximum dispensable volume of fluid.

### Visual Indicators

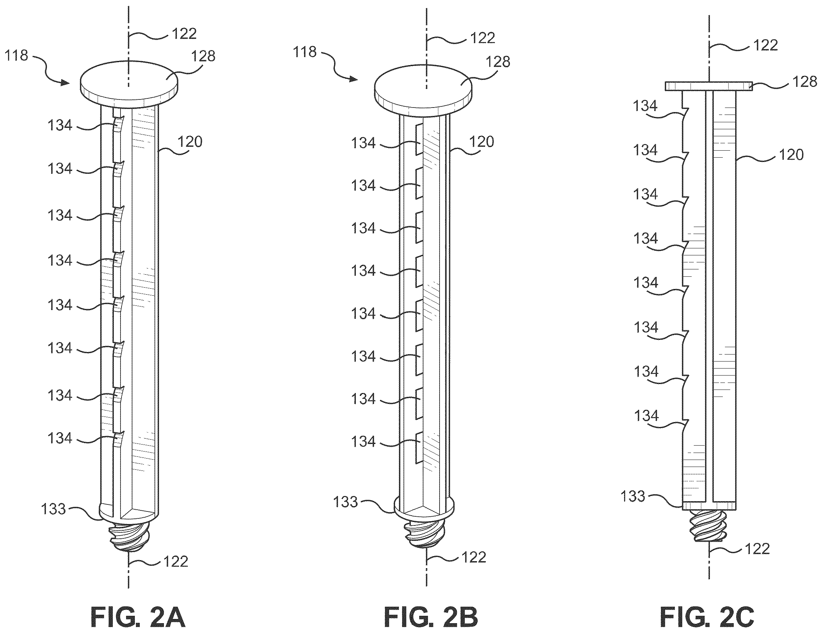

Referring to **FIGS. 2A-2C****,** the plunger rod 120 according to the first embodiment comprises a plurality of visual indicators **132** disposed along its length, each preferably corresponding to a clinically relevant volume of fluid. The visual indicators 132 may comprise printed, embossed, engraved, or otherwise formed markings on the plunger rod 120, and may include numerals indicating volume (e.g., in milliliters or cubic centimeters), color-coded bands, alphanumeric patient-weight or patient-size designations, or any combination thereof. In a preferred embodiment, the visual indicators 132 correspond to discrete, clinically relevant volume increments determined by patient-specific dosing parameters, such as weight-based dosing tables, body surface area calculations, or age-based dosing references commonly employed in emergency medicine and pediatric care.

The locking clip 130 described above is repeatedly positionable along the plunger rod 120 to align with any of the visual indicators 132, thereby setting a maximum dispensable volume of fluid corresponding to the clinically relevant dose volume indicated. In some embodiments, the barrel 104 comprises additional volume graduation markings on an external surface of the cylindrical body 106 (not shown), which may be used in conjunction with the visual indicators 132 on the plunger rod 120 to confirm the selected dose setting.

### Plunger Rod

With continued reference to FIGS. 2A-2C, the plunger rod 120 extends along the longitudinal axis 122 from the proximal thumb press 128 to a distal end **133** of the plunger rod. In the first embodiment, the thumb press 128 has a disc-shaped configuration providing a broad contact surface for the thumb or palm of a user. The disc-shaped thumb press 128 may be formed integrally with the plunger rod 120 or may be formed as a separate component attachable to a proximal end of the plunger rod 120.

The plunger rod 120 comprises a plurality of indentations **134** spaced longitudinally along its length. Each indentation 134 preferably corresponds to a different selected maximum dispensable volume of fluid. In addition, in preferred embodiments, each indentation 134 also corresponds to a clinically relevant volume increment of fluid. The indentations 134 may be peripherally formed around the plunger rod 120, such as annular grooves, or may be formed on one or more specific faces or sides of the plunger rod 120. In an embodiment, the plunger rod 120 has a cross-shaped, H-shaped, or multi-vaned cross-sectional profile, and the indentations 134 are formed as transverse notches or depressions in one or more of the vanes or faces of the plunger rod 120. The shape and depth of each indentation 134 are configured to receive and engage corresponding features of the locking clip 130, as further described herein. The spacing between adjacent indentations 134 is preferably selected to correspond to the desired volume increments, based on the cross-sectional area of the barrel 104 interior chamber 108. As such, the indentations 134 may be uniformly spaced apart from one another, non-uniformly spaced apart from one another, or a mixture of uniform and non-uniformly spaced apart from one another.

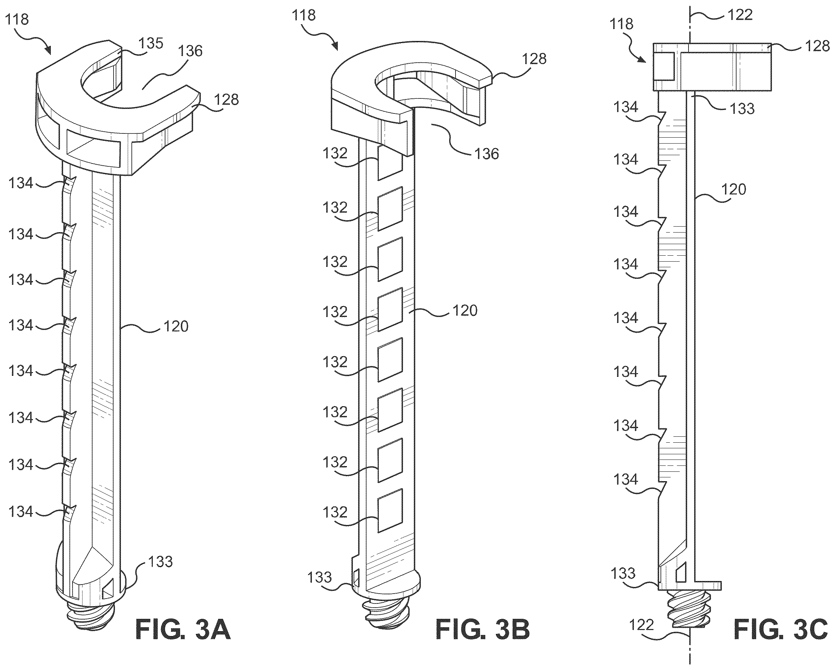

Referring to **FIGS. 3A-3C****,** a plunger rod 120 according to a second embodiment is shown. The second embodiment is similar to the first embodiment, except that the thumb press 128 has a different configuration. In the second embodiment, the thumb press 128 has a crescent-shaped configuration that provides an enlarged gripping surface and enables the plunger rod 120 to nest alongside the outer surface of the cylindrical body 106 of the barrel 104 during storage and transport. In addition, as described in U.S. Patent No. 12,440,623, which is incorporated herein by reference in its entirety, the thumb press 128 may have a thumb interface/plunger flange **135.** The flange 135 may include a semi-circular cutout **136** to nest against an outer surface of the cylindrical body 106 of the barrel 104 (FIGS. 1A and 1B) during storage and transport.

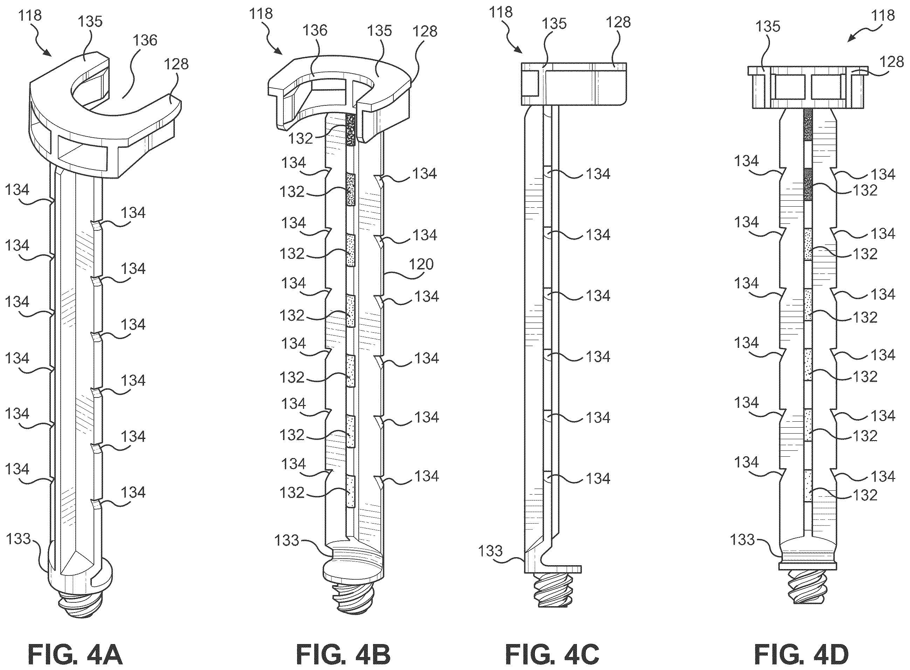

Referring now to **FIGS. 4A-4D****,** a plunger rod 120 according to a third embodiment is illustrated. The third embodiment is similar to the first and second embodiments, except that the thumb press 128 has a nested configuration that permits the plunger rod 120 to nest alongside the outer surface of the barrel 104 during storage and transport, and indentations 134 are located on each side surface of the plunger rod rather than the rear surface. As with the prior two embodiments, the thumb press 128 is oriented substantially perpendicular to the longitudinal axis 122 of the plunger rod 120, providing a broad surface for the user to apply pressure during fluid dispensing. This plunger rod 120 also includes visual indicators 132 on its surface that correspond to volume settings that may be established by the locking clip 130.

### Locking Clip

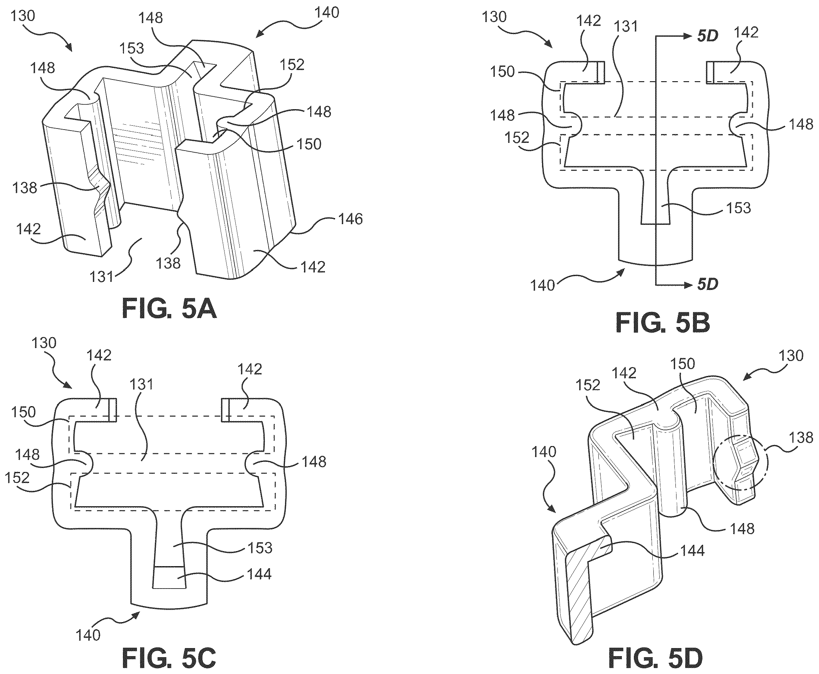

Turning now to **FIGS. 5A-5D****,** a first embodiment of the locking clip 130 is illustrated in detail. This embodiment may be referred to as a "push-type" locking clip due to its actuation mechanism, as will be described below. The locking clip 130 includes a body defining a central opening **131** through which the plunger rod 120 extends.

The locking clip 130 includes a pair of fluid volume indicators **138** extending inward from opposing lateral sides of the locking clip. Each fluid volume indicator 138 has an arrow-shaped configuration that points toward the plunger rod 120. The pair of fluid volume indicators 138 define a space or viewing window between their arrow-shaped tips through which the visual indicators 132 on the plunger rod 120 are visible. As the locking clip 130 is repositioned longitudinally along the plunger rod 120, the visual indicators 132 pass through the viewing window defined by the fluid volume indicators 138. When the locking clip 130 is positioned at a selected location, the arrow-shaped tips of the fluid volume indicators 138 point to and frame a corresponding one of the visual indicators 132, thereby enabling a user to visually confirm the maximum dispensable volume of fluid that will be delivered when the plunger 118 is fully advanced distally. This arrow-shaped configuration provides clear visual alignment and facilitates rapid, accurate dose setting.

The locking clip 130 further includes a gripping portion **140** that facilitates manual manipulation by the user. The gripping portion 140 may include textured surfaces, knurling, ribs, or other tactile features to enhance grip, particularly in wet or gloved-hand conditions. As shown in the section view of FIG. 5D, the locking clip 130 includes a pair of flexible arms **142** that extend away from the gripping portion 140 and extend vertically on opposing lateral sides of the locking clip.

Each flexible arm 142 includes a vertically-oriented rail **148** on its inside surface. Each rail 148 has a rounded inner surface that separates the central opening 131 into two distinct opening sections: a front opening section **150** located proximate to the space between the fluid volume indicators 138 at the front of the locking clip 130, and a rear opening section **152** located proximate to the gripping portion 140 where the user holds the locking clip 130.

The push-type locking clip 130 operates via a bistable push-pull actuation mechanism. When the user pulls the locking clip 130 rearwards away from the plunger rod 120, the flexible arms 142 flex outward, and the plunger rod 120 snaps into the front opening section 150. In this released configuration, the plunger rod 120 is held loosely within the front opening section 150, allowing the locking clip 130 to slide freely along the longitudinal axis 122 of the plunger rod 120 to select a desired dose position. The fluid volume indicators 138, in addition to providing visual alignment, extend sufficiently inward to help retain the plunger rod 120 within the front opening section 150 during repositioning. To permit the above-described functionality, in preferred embodiments, the material of the locking clip 130 may be a resilient polymer such as polypropylene, polyethylene, or a thermoplastic elastomer that provides suitable flexibility for the bistable snap action and elastic recovery of the flexible arms 142.

When the locking clip 130 is pushed forward toward the plunger rod 120, the flexible arms 142 flex and the plunger rod 120 snaps into the rear opening section 152. A vertically-oriented rear slot **153** is provided between flexible arms 142 and extends rearwardly from the rear opening section 152. The rear slot 153 is sized and configured to receive a portion of the plunger rod 120 and to guide the locking clip 130 as it slides vertically along the plunger rod. A forwardly-extending rear projection **144** is positioned within the rear slot 153 and projects toward the front opening between the flexible arms 142. When the plunger rod 120 is received in the rear opening section 152 and the locking clip 130 is slid longitudinally along the plunger rod 120 to align with a desired position, the inward projection 144 engages one of the indentations 134 on the plunger rod 120, thereby securely locking the locking clip 130 at the selected position.

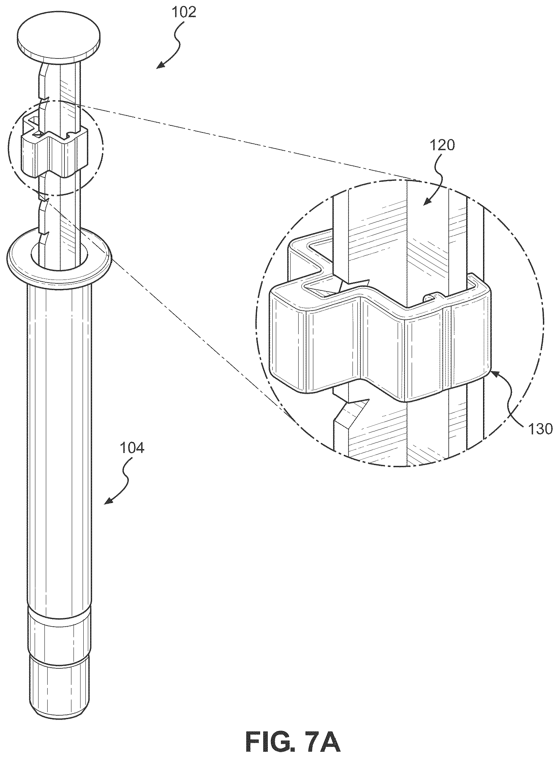

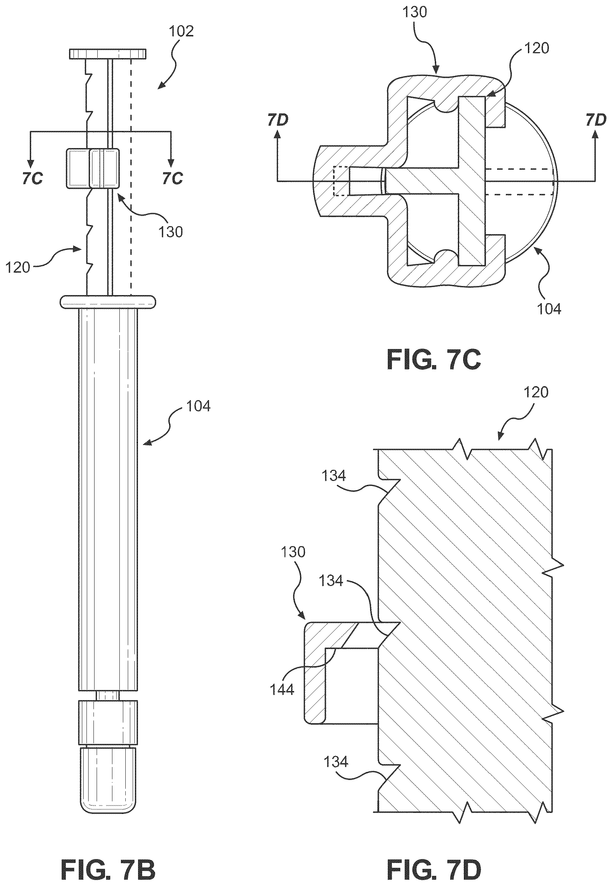

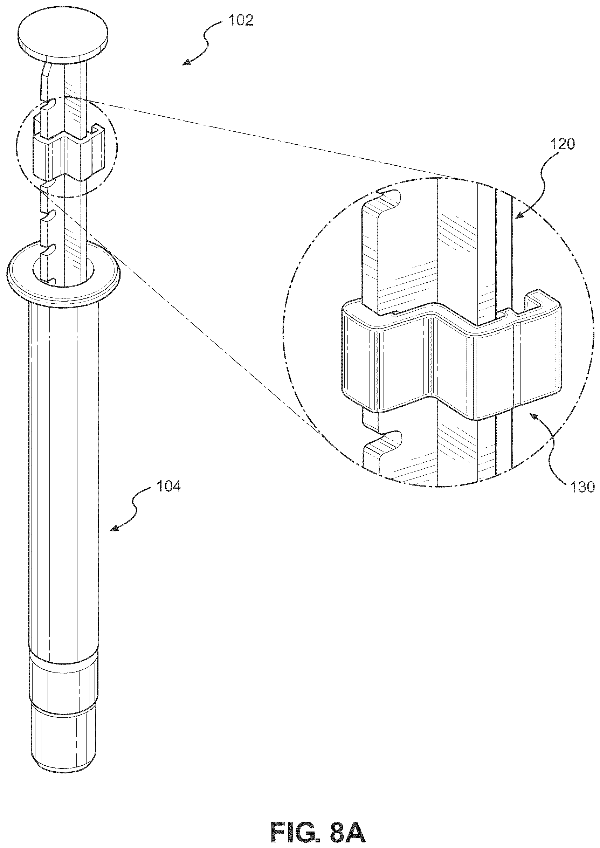

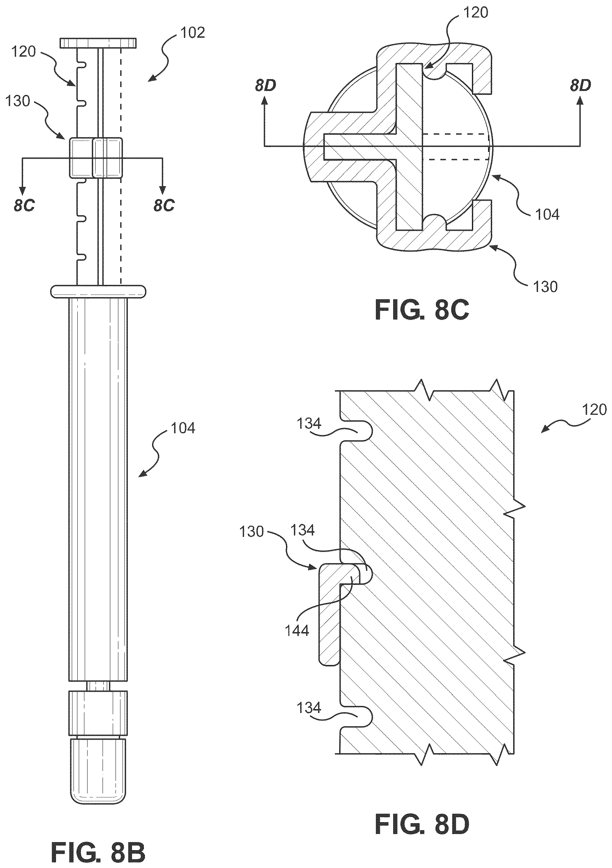

In this embodiment, the indentations 134 on the plunger rods 120 shown in FIGS. 2A-2C and 3A-3C comprise rear or central indentations having a triangular or V-shaped cross-sectional geometry. However, in other embodiments, other shapes, including rounded shapes, may be used in place of the triangular or V-shaped cross-sectional geometry. For example, FIGS. 7A-7D illustrate a syringe assembly employing indentations 134 having a triangular cross-sectional geometry, while FIGS. 8A-8D illustrate a syringe assembly employing indentations 134 having a rounded cross-sectional geometry. In each case, the engagement of the rear projection 144 with one of the indentations 134 provides positive engagement between the locking clip 130 and the plunger rod 120 in the locked configuration, thereby resisting unintended displacement of the locking clip 130 during storage, transport, or fluid dispensing. At the same time, however, pulling the locking clip 130 rearwards permits easy longitudinal sliding movement of the locking clip along plunger rod 120 in the released configuration. In each case, the bistable snap mechanism provides tactile feedback to the user, confirming the transition between released and locked configurations. It is noted that, in FIGS. 7B, 7C, 8B, and 8C, dashed lines are used to illustrate an additional plunger rod rib that may be present depending on the rib configuration employed. It will be appreciated by those of skill in the art that plunger rods having 3 ribs, 4 ribs, or other rib or plunger rod configurations may be used.

The push-type locking clip 130 shown in FIGS. 5A-5D is configured for use with the plunger rods of the first and second embodiments (FIGS. 2A-2C and 3A-3C) due to the configuration of the rear or central indentations 134 and the corresponding position of the inward projection 144. However, other configurations of push-type locking clips may be adapted for use with other plunger rod configurations

Finally, the locking clip 130 also includes one or more stop surfaces **146** that are positioned to abut against the outer rim 116 of the proximal entrance 114 of the barrel 104 when the plunger 118 is advanced to the maximum dispensable volume corresponding to the position of the locking clip 130. More particularly, in preferred embodiments, the locking clip 130 is sized such that its overall outer dimensions exceed the inner diameter of the proximal entrance 114 of the barrel 104. Accordingly, a bottom surface 146 of the locking clip 130, optionally including one or more of the bottom surfaces of both the grip portion 140 and the flexible arms 142, extends over and contacts the outer rim 116 of the proximal entrance 114 when the plunger 118 is fully advanced distally, thereby limiting further longitudinal movement of the plunger 118 with respect to the barrel 104.

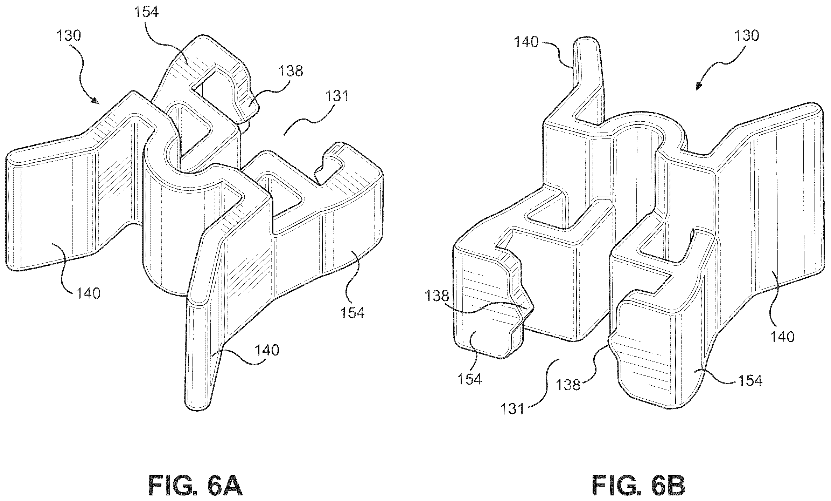

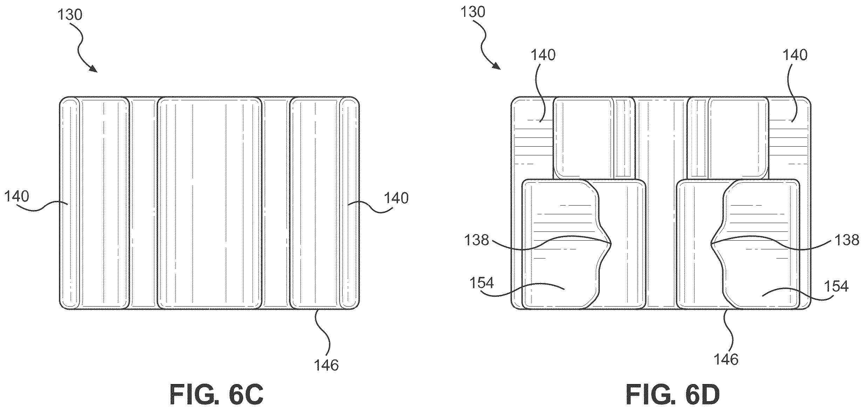

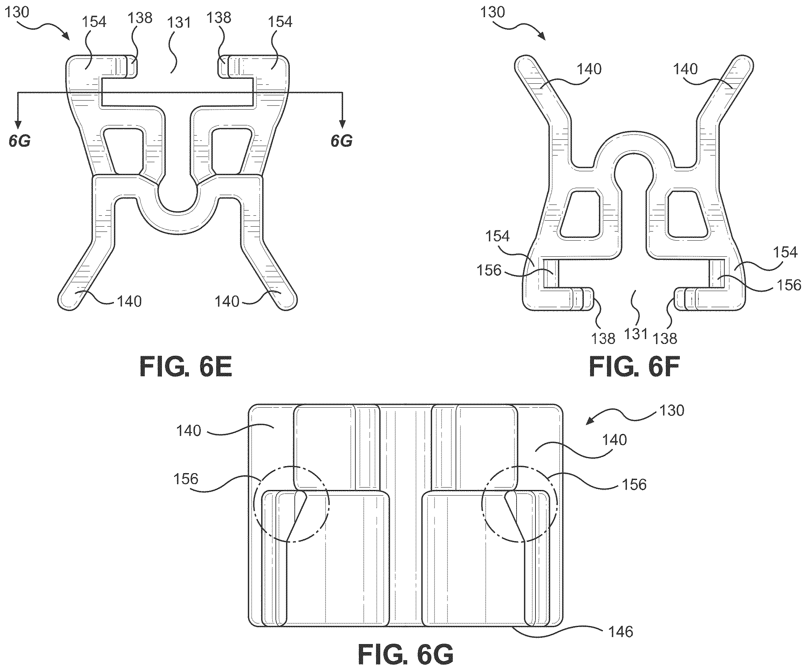

Referring now to **FIGS. 6A-6G****,** a second embodiment of the locking clip 130 is illustrated. This embodiment may be referred to as a "butterfly-type" locking clip due to its bilateral wing-like arms and squeeze-to-release actuation mechanism. The butterfly-type locking clip 130 also includes a gripping portion 140 that facilitates manual manipulation by the user. The gripping portion 140 may include textured surfaces, knurling, ribs, or other tactile features to enhance grip. Like the push-type locking clip 130 described above, the locking clip 130 also includes a pair of fluid volume indicators **138** extending inward from opposing lateral sides of the locking clip.

Additionally, the butterfly-type locking clip 130 comprises a pair of bilateral wing-like arms **154** extending outward from opposing lateral sides of the locking clip. Each wing-like arm 154 includes an inward projection **156** that extends toward the plunger rod 120. The locking clip 130 also includes a body defining a central opening 131 through which the plunger rod 120 extends. However, unlike the push-type locking clip described above, the butterfly-type locking clip 130 defines a single central opening section 131 through which the plunger rod 120 extends instead of a divided (i.e., 2-part) central opening section. In the illustrated butterfly-type locking clip 130, the inward projections 156 replace the vertically-oriented rails 148 of the push-type clip and are positioned to engage the peripheral indentations 134 on the plunger rod 120 shown in FIGS. 4A-4D. As such, the butterfly-type locking clip 130 is configured for use with the third plunger rod embodiment shown in FIGS. 4A-4D, which includes peripheral indentations 134 positioned on opposing lateral edges of the plunger rod 120.

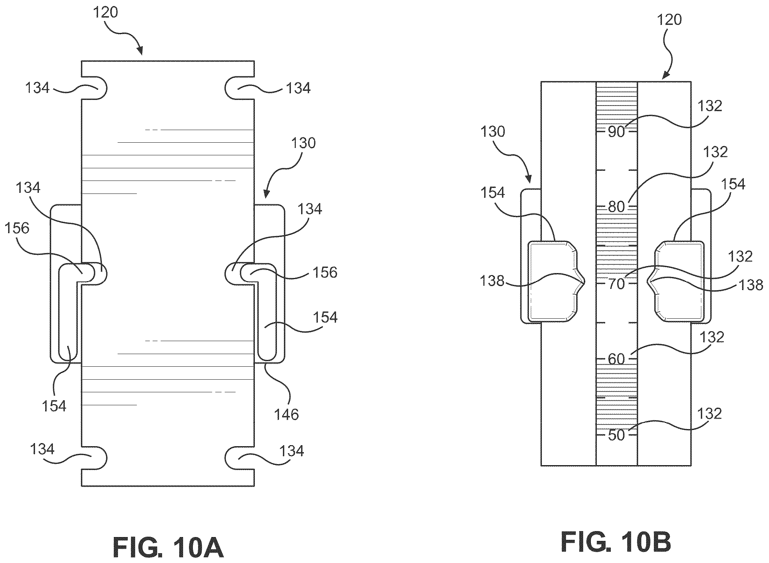

The butterfly-type locking clip 130 operates via a squeeze-to-release actuation mechanism. In a locked configuration, the wing-like arms 154 are in their nominal position, and the inward projections 156 automatically engage a pair of opposing peripheral indentations 134 on the plunger rod 120, as shown in **FIG. 10A****.** In this locked configuration, the locking clip 130 is securely held at the selected position and cannot be (or at least resists being) inadvertently moved.

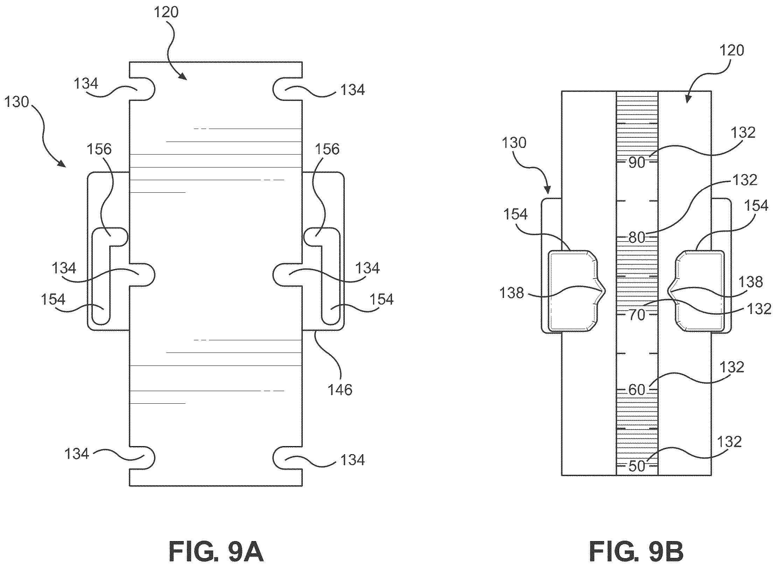

To reposition the locking clip 130, the user manually squeezes the rear grip portions 140 inward toward one another. This squeezing action causes the wing-like arms 154 to flex, disengaging the inward projections 156 outwards from the peripheral indentations 134. In this released configuration, shown in FIG. 9A, the locking clip 130 can be slid freely along the longitudinal axis 122 of the plunger rod 120 to a new position. When the user releases the squeezing pressure, the wing-like arms 154 automatically return to their nominal position due to the elastic memory of the material, and the inward projections 156 engage the nearest pair of peripheral indentations 134, thereby locking the clip 130 in place.

The butterfly-type locking clip 130 also includes one or more stop surfaces 146 that are positioned to abut against the outer rim 116 of the proximal entrance 114 of the barrel 104 when the plunger 118 is advanced to the maximum dispensable volume corresponding to the position of the locking clip 130. Similar to the push-type locking clip, the material of the butterfly-type locking clip 130 may be a resilient polymer such as polypropylene, polyethylene, or a thermoplastic elastomer.

Further, referring to FIGS. 9B and 10B, as with the push-type locking clip, the butterfly-type locking clip 130 includes a pair of fluid volume indicators 138 having an arrow-shaped configuration that extends inward from opposing lateral sides of the locking clip toward the plunger rod 120. The fluid volume indicators 138 define a viewing window through which the visual indicators 132 on the plunger rod 120 are visible, enabling the user to visually confirm the selected maximum dispensable volume.

In FIG. 9A and 9B, the butterfly-type locking clip 130 is shown in a configuration where the inward projections 156 are not fully engaged with the peripheral indentations 134. As shown, when the locking clip 130 is positioned such that the inward projections 156 are not aligned with a pair of peripheral indentations 134, the fluid volume indicator 138 is located between a pair of visual indicators 132 and is not aligned with either visual indicator. This visual misalignment provides feedback to the user that the locking clip 130 is not properly locked at a discrete, clinically relevant dose position.

Now, in FIGS. 10A and 10B, the butterfly-type locking clip 130 is shown in a fully locked and aligned configuration, where the inward projections 156 are fully engaged with a pair of peripheral indentations 134. When the locking clip 130 is positioned such that the inward projections 156 are fully engaged with a pair of peripheral indentations 134, the fluid volume indicator 138 is aligned with one of the visual indicators 132. This visual alignment confirms to the user that the locking clip 130 is properly locked at a discrete, clinically relevant dose position corresponding to the visual indicator 132.

In certain embodiments, if the locking clip 130 is initially positioned such that the inward projections 156 (in the case of the butterfly-type clip) or the inward projection 144 (in the case of the push-type clip) are not fully aligned with the indentations 134, the locking clip 130 is configured to automatically slide to and engage the nearest indentation 134 when the stop surface 146 contacts the outer rim 116 and dispensing force is continuously applied by the user. This automatic alignment feature ensures that the locking clip 130 settles into a discrete, repeatable position corresponding to one of the clinically relevant dose increments, even if the user's initial manual positioning was slightly imprecise. The automatic alignment is facilitated by the elastic flexibility of the locking clip material, the geometry of the indentations 134, and the force transfer that occurs when the stop surface contacts the outer rim 116 during plunger advancement.

### Syringe Assembly

Referring again to FIGS. 7A-7D, the syringe assembly 102 is shown with the plunger rod 120 from the first embodiment (FIGS. 2A-2C) and the push-type locking clip 130 in a released configuration. FIG. 7A provides a rear perspective view, while FIG. 7B shows a left elevation view. FIGS. 7C and 7D provide section views taken along lines 7C-7C and 7D-7D, respectively.

In the released configuration illustrated in these figures, the locking clip 130 has been pulled away from the plunger rod 120, causing the flexible arms 142 to flex outward and the plunger rod 120 to snap into the front opening section 150. The section view of FIG. 7D clearly shows the triangular cross-sectional geometry of the indentations 134 on the plunger rod 120. In the released configuration, the plunger rod 120 is positioned in the front opening section 150, and the locking clip 130 is free to slide longitudinally along the plunger rod 120. The user can freely reposition the locking clip 130 along the longitudinal axis 122 of the plunger rod 120 to select a different maximum dispensable volume by aligning the fluid volume indicators 138 with one of the visual indicators 132 on the plunger rod 120. The spacing of the indentations 134 corresponds to clinically relevant volume increments, ensuring that the locking clip 130 can be positioned to deliver precise, pre-set doses suitable for patient-specific requirements.

On the other hand, turning to FIGS. 8A-8D, the syringe assembly 102 is shown with the push-type locking clip 130 in a locked configuration. As in the previous figures, FIG. 8A is a rear perspective view, FIG. 8B is a left elevation view, and FIGS. 8C and 8D are section views taken along lines 8C-8C and 8D-8D, respectively. In the locked configuration, the locking clip 130 has been pushed toward the plunger rod 120, causing the flexible arms 142 to flex and the plunger rod 120 to snap into the rear opening section 152. The inward projection 144 is fully engaged within one of the indentations 134, securely holding the locking clip 130 at the selected position on the plunger rod 120. The locking clip 130 cannot be (or cannot easily be) inadvertently moved without the user intentionally actuating the release mechanism by pulling the locking clip 130 outward.

When the user advances the plunger 118 distally along the longitudinal axis 122 to dispense fluid 110 from the barrel 104, the plunger rod 120 and the locking clip 130 advance together distally toward the distal end 112, with the locking clip 130 remaining stationary relative to the plunger rod. When the maximum dispensable volume is reached, the stop surface 146 of the locking clip 130 contacts the outer rim 116 of the barrel 104. This physical contact between the stop surface 146 and the outer rim 116 provides tactile feedback to the user, signaling that the pre-set dose limit has been reached. Further advancement of the plunger rod 120 is prevented, ensuring that the precise volume corresponding to the position of the locking clip 130 is dispensed and preventing over-dosing. This feature is particularly valuable in emergency or high-stress situations where rapid, accurate dosing is critical.

### Method of Use

In a typical operational workflow using the syringe assembly 102 described herein, a user might first assess patient-specific parameters such as body weight, age, or body surface area to determine the appropriate fluid dose. The provider then positions the locking clip 130 on the plunger rod 120 by transitioning the locking clip to the released configuration (either by pulling the push-type clip away from the plunger rod, or by squeezing the butterfly-type clip inward), sliding the locking clip longitudinally along the plunger rod 120, preferably until the fluid volume indicators 138 align with the visual indicator 132 corresponding to the desired dose, and then transitioning the locking clip to the locked configuration (either by pushing the push-type clip toward the plunger rod, or by releasing the squeezing pressure on the butterfly-type clip).

If the syringe assembly 102 is provided as a prefilled syringe, the barrel 104 already contains the total volume of fluid 110. If the syringe assembly 102 is provided as an empty syringe, the provider aspirates the appropriate total volume of fluid 110 into the barrel 104 through the distal end 112 by retracting the plunger 118. Once the locking clip 130 is positioned and locked, the provider advances the plunger 118 distally to dispense the fluid 110 through the distal end 112. The stop surface 146 of the locking clip 130 contacts the outer rim 116 when the pre-set maximum dispensable volume has been delivered, providing tactile feedback and preventing further dispensing.

If the clinical situation requires administration of an additional dose (for example, if the patient does not respond adequately to the initial dose), the provider can reposition the locking clip 130 to a new position corresponding to a higher total dose without requiring a new syringe. This is accomplished by transitioning the locking clip 130 back to the released configuration, sliding the locking clip to the new position, and re-locking. This capability for rapid re-dosing is particularly valuable in emergency resuscitation scenarios where time is critical and multiple incremental doses may be required.

### Other Embodiments and Variations

In some embodiments, the visual indicators 132 on the plunger rod 120 are color-coded to correspond to different patient weight ranges, heights, or age groups. For example, a first color (e.g., blue) may indicate doses appropriate for neonates, a second color (e.g., green) may indicate doses for infants, a third color (e.g., yellow) may indicate doses for small children, and a fourth color (e.g., red) may indicate doses for larger children or adults. This color-coding system enables rapid visual identification of the appropriate dose range and reduces the risk of dosing errors.

In certain embodiments, the syringe assembly 102 is provided as part of a kit comprising multiple prefilled syringes, each containing a different medication commonly used in emergency resuscitation (e.g., epinephrine, atropine, amiodarone, naloxone, etc.). Each syringe in the kit is provided with a locking clip 130 already mounted on the plunger rod 120, enabling the healthcare provider to rapidly select and administer the appropriate dose for each medication based on patient-specific parameters.

In some embodiments, the barrel 104 and plunger rod 120 are transparent or translucent, and the locking clip 130 is fabricated from a contrasting opaque or colored material to enhance visual distinction. In other embodiments, the locking clip 130 is fabricated from a transparent or translucent material with color-coded portions corresponding to different dose ranges.

In certain embodiments, the plunger rod 120 includes alphanumeric designations in addition to or in place of numeric volume markings. For example, the visual indicators 132 may include patient weight ranges (e.g., "5-10 kg", "10-15 kg", etc.), patient age ranges (e.g., "0-6 months", "6-12 months", etc.), or other clinically relevant parameters.

Although the embodiments described above illustrate two specific locking clip configurations (push-type and butterfly-type), it will be appreciated by those skilled in the art that other locking clip configurations employing different grip mechanisms and actuation methods may be used without departing from the scope of the present invention, provided that such configurations enable longitudinal repositioning of the locking clip along the plunger rod without rotation, provide secure engagement in a locked configuration, and include a stop surface that limits plunger travel by contacting the outer rim of the barrel.

The syringe assembly 102 described herein is compatible with standard manufacturing processes for medical syringes and can be fabricated using conventional injection molding, extrusion, and assembly techniques. The locking clip 130 may be provided as a separate component that is manually installed onto the plunger rod 120 by the e nd user, or may be pre-installed during manufacturing. In some embodiments, multiple locking clips of different sizes or configurations are provided with a single syringe, allowing the user to select the locking clip best suited to the specific clinical application.

Although this description contains many specifics, these should not be construed as limiting the scope of the invention but as merely providing illustrations of some of the presently preferred embodiments thereof, as well as the best mode contemplated by the inventor of carrying out the invention. The invention, as described herein, is susceptible to various modifications and adaptations as would be appreciated by those having ordinary skill in the art to which the invention relates.

## Claims

1. A dosing plunger system for a precise dosing syringe assembly having a barrel formed by a cylindrical body with an interior chamber configured to contain a total volume of fluid, a distal end for dispensing the fluid contained in the interior chamber, and a proximal entrance having an outer rim, the system comprising:
a plunger having a plunger rod extending along a longitudinal axis and configured to move within the barrel;
a locking clip mounted on the plunger rod, the locking clip configured to slide longitudinally along the plunger rod without rotation relative to the plunger rod to a selected position, the locking clip comprising:
a gripping portion configured to be manually actuated by a user to transition the locking clip between a locked configuration and a released configuration;
one or more engagement features configured to engage the plunger rod in the locked configuration to prevent longitudinal movement of the locking clip relative to the plunger rod, and to disengage from the plunger rod in the released configuration to permit the locking clip to slide longitudinally along the plunger rod; and
a stop surface sized such that an outer extent thereof exceeds the inner diameter of the proximal entrance of the barrel,
wherein, when the plunger is pushed distally, (1) the one or more engagement features engage the plunger rod to prevent further longitudinal movement of the locking clip relative to the plunger rod in the locked configuration, thereby maintaining the selected position, and (2) the stop surface contacts the outer rim of the proximal entrance of the barrel, thereby limiting longitudinal movement of the plunger with respect to the barrel,
wherein the locking clip is repeatedly repositionable to different selected positions along the plunger rod without requiring rotation of the locking clip relative to the plunger rod, and
wherein the selected position of the locking clip along the plunger rod determines a selected maximum dispensable volume of fluid dispensed from the interior chamber when the plunger is fully advanced distally until the stop surface contacts the outer rim of the proximal entrance of the barrel.

2. The dosing plunger system of claim 1, wherein the gripping portion comprises at least two grip portions that are squeezable inward to transition the locking clip to the released configuration, thereby causing the one or more engagement features to disengage from the plunger rod, and wherein the one or more engagement features automatically re-engage the plunger rod in the locked configuration when squeezing pressure is released.

3. The dosing plunger system of claim 2, wherein the gripping portion comprises a pair of bilateral wing-like arms extending outward from opposing lateral sides of the locking clip, each wing-like arm comprising an inward projection configured to engage a corresponding peripheral indentation on the plunger rod in the locked configuration and to disengage from the peripheral indentation when the wing-like arms are squeezed inward.

4. The dosing plunger system of claim 1, wherein the gripping portion comprises a push-actuated mechanism configured to transition the one or more engagement features to the locked configuration when the gripping portion is pressed toward the plunger rod and to transition the one or more engagement features to the released configuration when the gripping portion is pulled away from the plunger rod.

5. The dosing plunger system of claim 1, wherein the plunger rod comprises a plurality of indentations spaced longitudinally along the plunger rod that each correspond to a different selected maximum dispensable volume of fluid, and wherein the one or more engagement features of the locking clip are configured to engage one of the plurality of indentations when positioned at the selected position.

6. The dosing plunger system of claim 5, wherein each indentation of the plurality of indentations corresponds to a clinically relevant volume increment.

7. The dosing plunger system of claim 1, wherein the stop surface is formed by a bottom surface of the locking clip, the bottom surface sized such that an outer extent thereof exceeds the inner diameter of the proximal entrance of the barrel, whereby the bottom surface contacts the outer rim when the plunger is fully advanced distally.

8. The dosing plunger system of claim 1, wherein the plunger rod comprises visual indicators each corresponding to a clinically relevant volume of fluid, and wherein the locking clip is repeatedly positionable to align with any of the visual indicators to set a maximum dispensable volume of fluid corresponding to the clinically relevant volume of fluid.

9. The dosing plunger system of claim 1, wherein the one or more engagement features comprise projections extending inward toward the plunger rod to engage the plunger rod in the locked configuration.

10. The dosing plunger system of claim 1, wherein the locking clip further comprises tactile grip features on an external surface of the gripping portion to facilitate manual manipulation of the locking clip by a user.

11. The dosing plunger system of claim 1, wherein the plunger further comprises a proximal thumb press configured to be manually actuated by a user to advance the plunger distally within the barrel.

12. A syringe assembly for pre-setting a precise fluid dose comprising:
a barrel formed by a cylindrical body having an interior chamber configured to contain a total volume of fluid, a distal end for dispensing the fluid contained in the interior chamber, and a proximal entrance having an outer rim;
a plunger comprising a plunger rod having a proximal thumb press; and
a repositionable locking clip mounted on the plunger rod between the thumb press and the proximal entrance of the barrel, the locking clip comprising:
a gripping portion configured to be manually actuated by a user to transition the locking clip between a locked configuration and a released configuration;
one or more engagement features configured to engage the plunger rod in the locked configuration to prevent sliding movement of the locking clip along the plunger rod, and to disengage from the plunger rod in the released configuration to permit sliding movement of the locking clip along the plunger rod; and
a stop surface sized such that an outer extent thereof exceeds the inner diameter of the proximal entrance of the barrel,
wherein the locking clip is repeatedly repositionable to different selected positions along the plunger rod at any time after the barrel contains fluid and without requiring rotation of the locking clip relative to the plunger rod, such that each selected position determines a selected maximum dispensable volume when the plunger is subsequently advanced distally until the stop surface contacts the outer rim of the proximal entrance during fluid dispensing,
wherein, when the plunger is advanced distally during fluid dispensing and the stop surface contacts the outer rim of the proximal entrance, the one or more engagement features engage the plunger rod to prevent further longitudinal movement of the locking clip relative to the plunger rod, thereby limiting the dispensed volume to the selected maximum dispensable volume of fluid, and
wherein the barrel is configured to receive fluid either by aspiration through the distal end or as a prefilled syringe.

13. The syringe assembly of claim 12, wherein the gripping portion comprises at least two grip portions that are squeezable inward to transition the locking clip to the released configuration, thereby causing the one or more engagement features to disengage from the plunger rod, and wherein the one or more engagement features automatically re-engage the plunger rod when squeezing pressure is released, or wherein the gripping portion comprises a push-actuated mechanism configured to transition the one or more engagement features to the locked configuration when pressed toward the plunger rod and to transition the one or more engagement features to the released configuration when pulled away from the plunger rod.

14. The syringe assembly of claim 12, wherein the plunger rod comprises a series of indentations spaced longitudinally along the plunger rod, and wherein the one or more engagement features engage at least one of the indentations when in the locked configuration.

15. The syringe assembly of claim 12, wherein the stop surface is formed by a bottom surface of the locking clip, the bottom surface sized such that an outer extent thereof exceeds the inner diameter of the proximal entrance of the barrel, whereby the bottom surface contacts the outer rim when the plunger is fully advanced distally.

16. A method of preparing and administering a precise fluid dose using a syringe assembly, the method comprising:
(a) providing a syringe assembly comprising:
a barrel having a proximal entrance with an outer rim and filled with fluid;
a plunger with a plunger rod extending proximally from the barrel, the plunger rod comprising a plurality of indentations spaced longitudinally along the plunger rod; and
a locking clip mounted on the plunger rod proximal to the barrel;
(b) selecting a maximum dispensable volume of fluid based on patient parameters;
(c) positioning the locking clip on the plunger rod at a selected position corresponding to the maximum dispensable volume of fluid by sliding the locking clip longitudinally along the plunger rod without rotating the locking clip, such that a portion of the locking clip will contact the outer rim of the proximal entrance of the barrel when the plunger is advanced to a position corresponding to dispensing the maximum dispensable volume of fluid; and
(d) advancing the plunger distally to dispense the fluid, wherein a dispensed volume does not exceed the maximum dispensable volume of fluid.

17. The method of claim 16, wherein step (c) comprises manually squeezing a gripping portion of the locking clip to reduce frictional engagement with the plunger rod, sliding the locking clip to the selected position, and releasing the gripping portion to automatically increase frictional engagement with the plunger rod, or wherein step (c) comprises pulling the locking clip away from the plunger rod to transition the locking clip to a released configuration, sliding the locking clip to the selected position, and pressing the locking clip toward the plunger rod to transition the locking clip to a locked configuration.

18. The method of claim 16 wherein, if the locking clip is not initially aligned with one of the plurality of indentations, the locking clip automatically slides to and engages a nearest one of the plurality of indentations when the portion of the locking clip contacts the outer rim and dispensing force is continuously applied.
